Europäisches Patentamt

European Patent Office

Office européen des brevets

(19)

(11) Numéro de publication: **0 226 487**
**B1**

(12) **FASCICULE DE BREVET EUROPEEN**

(45) Date de publication du fascicule du brevet:
**10.01.90**

(51) Int. Cl.⁴: **C07C 2/76**, B01J 19/24

(21) Numéro de dépôt: **86402474.0**

(22) Date de dépôt: **04.11.86**

(54) Procédé de conversion thermique du méthane en hydrocarbures de poids moléculaires plus élevés.

(30) Priorité: 08.11.85 FR 8516714
18.03.86 FR 8603970
23.06.86 FR 8609168

(43) Date de publication de la demande:
**24.06.87 Bulletin 87/26**

(45) Mention de la délivrance du brevet:
**10.01.90 Bulletin 90/2**

(84) Etats contractants désignés:
**DE GB IT NL**

(56) Documents cités:
**WO-A-85/00164**
**DE-A- 1 542 406**
**FR-A- 711 394**
**FR-A- 1 364 835**

(73) Titulaire: **INSTITUT FRANCAIS DU PETROLE, 4, Avenue de Bois-Préau, F-92502 Ruell-Malmaison(FR)**

(72) Inventeur: **Alagy, Jacques, 24, Chemin Beckensteiner, F-69260 Charbonnières(FR)**
Inventeur: **Busson, Christian, 11, Chemin du Cogny, F-69570 Dardilly(FR)**

**Description**

L'invention concerne un procédé amélioré de conversion thermique du méthane en hydrocarbures de poids moléculaires plus élevés.

Parmi les sources de méthane se trouvent les gaz naturels et les gaz de raffinerie. Les gaz naturels peuvent être où non des gaz associés à de l'huile brute ; leur composition varie de façon assez sensible suivant leur provenance, mais ils contiennent généralement un pourcentage volumique de méthane compris entre 60 et 95 %. Ce méthane est toujours associé à d'autres alcanes supérieurs, pouvant atteindre et même dépasser des hydrocarbures en $C_6$. Divers procédés cryogéniques permettent de séparer en plusieurs fractions les gaz une fois débarassés de l'eau et des composants acides : azote, gaz naturels liquéfiés dont on sépare la fraction propane et butane, et une fraction composée essentiellement de méthane associé à une faible quantité d'éthane. Cette dernière fraction est soit réinjectée dans le puits pour maintenir la pression qui fait monter le pétrole brut, soit expédiée par gazoduc comme gaz combustible, soit encore brûlée à la torche.

D'autres sources de méthane sont les gaz de raffinerie qui sont d'origine multiple : gaz de première distillation du pétrole brut, gaz d'hydroréformage, d'hydrotraitements divers, gaz de craquage thermique, gaz de craquage catalytique ; tous ces gaz contiennent, dans des proportions diverses, du méthane associé à de nombreux autres composants gazeux, tels que des hydrocarbures légers, de l'azote, du $CO_2$.

C'est ainsi, par exemple qu'un gaz effluent d'une unité de craquage catalytique en lit fluidisé comprend, après lavage, de l'ordre de 30 % en volume de méthane. Ce gaz est souvent fractionné par refroidissement sous pression, permettant d'obtenir deux fractions, l'une comprenant de l'hydrogène, de l'azote, du méthane et une faible partie d'éthylène, l'autre fraction étant composée de la majeure partie de l'éthylène initial, d'éthane, de propane, de propylène. Cette dernière fraction peut être avantageusement envoyée à une unité de type Dimersol, alors que la première est retournée vers le réseau de fuelgaz de la raffinerie où elle est utilisée comme combustible.

La conversion de méthane en hydrocarbures de poids moléculaires plus élevés présente donc un intérêt certain ; c'est ainsi que, dans les gisements lointains de gaz naturels ou de gaz associés, la conversion du méthane en acétylène, éthylène et aromatiques peut permettre, par l'utilisation d'enchainements de procédés connus, d'obtenir des fractions liquides plus facilement transportables et/ou valorisables.

Par exemple, après séparation des solides formés éventuellement, on peut séparer la fraction de composés aromatiques, puis traiter la fraction gazeuse tout d'abord dans des unités permettant l'oligomérisation et/ou la cyclisation de l'acétylène, et ensuite, après une nouvelle séparation gaz/liquides, traiter la fraction gazeuse résiduelle, riche en éthylène, dans des unités de type Dimersol, qui permettent d'obtenir des oligomères de l'éthylène.

Sur les lieux mêmes de raffinage, la conversion, même partielle, du méthane en produits plus facilement valorisables présente également un grand intérêt économique.

Il a déjà été proposé différentes méthodes de conversion du méthane ; c'est ainsi que le brevet US 4.199.533 décrit une méthode d'obtention d'éthylène et/ou d'éthane à partir de méthane, consistant à faire réagir sur ce produit du chlore à température supérieure à 700 °C. Ce procédé présente l'inconvénient important de mise en oeuvre à haute température de gaz très corrosifs, comme le chlore et l'acide chlorhydrique.

Parmi les procédés de craquage thermique susceptibles de transformer du méthane, le procédé dit "procédé Wulff" consiste à utiliser des masses de contact réfractaires ; dans un premier temps on chauffe la masse réfractaire par combustion à l'air d'un combustible qui peut être constitué par la charge elle-même puis, dans un second temps, l'hydrocarbure à craquer est décomposé en absorbant de la chaleur emmagasinée par le matériau réfractaire lors de la période précédente ; il s'agit donc d'un procédé discontinu.

Les procédés à arc électrique et à plasma sont axés essentiellement sur la préparation d'acétylène ; leur consommation élevée en énergie électrique les rend difficilement exploitables.

Un troisième type de procédé, quelquefois nommé autothermique, consiste à brûler une partie de la charge pour fournir les calories nécessaires à la réaction de craquage ; ce type de procédé met en oeuvre un brûleur dans lequel environ 1/3 de l'hydrocarbure est brûlé, le reste étant craqué. Etant donné les hauts niveaux thermiques atteints, ce type de procédé produit essentiellement de l'acétylène et du coke.

Le brevet FR 1.211.695 décrit un procédé de pyrolyse combiné d'hydrocarbures consistant à mélanger du méthane à des gaz de combustion chauds ne comportant pas d'oxygène en excès, puis à injecter dans le mélange obtenu des hydrocarbures paraffiniques à plus d'un atome de carbone ; suivant ce procédé, une très faible partie du méthane peut être transformée en acétylène.

Il n'existe pas actuellement, de procédé industriel utilisant un transfert de chaleur contrôlé à travers une paroi permettant de transformer du méthane en hydrocarbures facilement valorisables, tels qu'acétylène, éthylène, et/ou composés aromatiques.

L'objet de cette invention est de remédier à cette lacune en proposant un procédé de conversion thermique du méthane en hydrocarbures de poids moléculaires plus élevés.

Plus particulièrement, l'invention réside en l'utilisation d'un système continu mutlicanaux réalisé en matière céramique, et préférentiellement en carbure de silicium, de section quelconque mais avantageusement ronde, carrée ou rectangulaire, composé d'une pluralité de canaux parallèles, formant des rangées, les canaux étant de section quelconque mais avantageusement polygonale et préférentiellement carrée ou rectangulaire, et conçu de telle façon que les rangées de canaux d'ordre ... n-2, n, n + 2, n + 4 ... soient parcourues par la charge à traiter et que les autres rangées de canaux d'ordre ...n-1, n + 1, n + 3, ... soient parcourues par des fluides caloporteurs ou réfrigérants, n'étant un nombre quelconque.

Peu importe le fluide choisi pour la première et la dernière rangée, l'essentiel étant de respecter l'alternance entre rangées.

Les rangées de canaux parcourus par la charge sont continues sur la longueur totale de l'ensemble. Les autres rangées de canaux sont fractionnées en deux parties successives par un cloisonnement en un point intermédiaire de leur longueur, de façon à définir deux zones, les canaux de la première zone (dans le sens d'écoulement de la charge à traiter) étant parcourus par un fluide caloporteur déterminant ainsi une zone de pyrolyse ou zone réactionnelle, les canaux de la deuxième zone (côté sortie de l'effluent) étant parcourus par un fluide réfrigérant déterminant ainsi une zone de refroidissement, ou zone de trempe.

Le système ainsi obtenu constitue donc un ensemble continu réacteur de pyrolyse et échangeur de trempe.

Dans la réalisation préférentielle de l'invention, l'arrivée du fluide caloporteur dans les rangées de canaux destinés à le véhiculer s'effectue sensiblement perpendiculairement à l'axe de ces rangées de canaux, au moyen d'une ouverture pratiquée dans l'une des parois latérales des canaux concernés situés à la périphérie, et les canaux d'une même rangée sont mis en communication au niveau de l'arrivée du fluide caloporteur par des ouvertures pratiquées dans leurs parois latérales, de façon à ce que la totalité des canaux destinés à cet usage soit parcourue par le fluide caloporteur.

De préférence, l'arrivée du fluide caloporteur au niveau de la zone de pyrolyse s'effectue en un point intermédiaire de cette zone, situé à une distance du début de cette zone (le début étant déterminé par l'endroit d'introduction de la charge) représentant 5 à 50 % de la longueur totale de cette zone, et plus préférentiellement 20 à 40 % de cette zone ; de cette façon, l'apport maximum de chaleur peut être transmis aux canaux parcourus par la charge à l'endroit où se produisent les réactions endothermiques de craquage et de déshydrogénation.

Le nombre total de rangées de canaux n'est pas déterminant dans le procédé ; il est évidemment fonction de la dimension de l'ensemble réacteur de pyrolyse-échangeur de trempe et des dimensions d'un canal unitaire. Cependant, les deux rangées de canaux extérieures sont, dans le cadre de l'invention, de préférence parcourues par les fluides caloporteurs ou réfrigérants.

Le nombre de canaux unitaires par rangée n'est pas non plus déterminant et est fonction de la dimension globale de l'ensemble et de la dimension d'un canal unitaire.

Un canal unitaire présente une section avantageusement comprise entre 9 et 900 mm² et préférentiellement comprise entre 25 et 100 mm² ; la longueur d'un canal unitaire peut être variable suivant les charges à traiter, la température du procédé, le temps de contact désiré et la température à laquelle on effectue la trempe. La longueur totale d'un canal unitaire - comprenant zone de pyrolyse et zone de trempe - est généralement comprise entre 2 et 15 m et, de préférence, entre 5 et 10 m.

Dans un mode particulier de réalisation du procédé, chaque canal unitaire peut être subdivisé en une pluralité de canaux élémentaires plus petits.

Dans le cadre de l'invention, on peut réaliser l'ensemble continu réacteur de pyrolyse - échangeur de trempe soit sous forme d'un monobloc, soit encore par juxtaposition jointive d'éléments unitaires, de forme identique, qui sont assemblés entre eux par tout moyen adéquat, comme par exemple à l'aide de brides. L'utilisation de céramique, et plus particulièrement du carbure de silicium, matériau facilement extrudable, rend facile la mise en oeuvre de tels ensembles ou éléments d'ensembles.

Les charges à traiter ont un temps de résidence dans la zone réactionnelle compris entre 5 et 1000 millisecondes, et plus avantageusement compris entre 200 et 500 millisecondes ; la température des parois dans la zone réactionnelle peut être portée à une valeur maximale de l'ordre de 1 500 °C.

Toute céramique réfractaire résistant à des températures supérieures à 1150 °C peut être utilisée dans le cadre de l'invention ; le carbure de silicium, qui présente une bonne conductibilité thermique et qui est facilement mis en oeuvre par extrusion est la céramique préférentiellement utilisée.

De nombreux échangeurs indirects de chaleur en céramique ont été décrits dans l'art antérieur ; leur domaine d'utilisation concerne essentiellement les moteurs à turbine, où le matériau de l'échangeur doit résister à des températures de l'ordre de 1 200 à 1 400 °C. On peut citer, par exemple, le brevet français 2 436 956, le brevet US 4 421 702, les demandes de brevets japonais 59 046 496 et 59 050 082, ainsi que les brevets français 2 414 988 et 2 436 958 (brevet d'addition du précédent), ce dernier brevet décrivant un procédé de fabrication d'un élément d'échange indirect de chaleur en matière céramique pouvant être avantageusement utilisé dans le cadre de l'invention, moyennant des aménagements.

Le procédé de l'invention permet de convertir thermiquement du méthane en acétylène, éthylène et produits benzéniques, essentiellement, grâce aux caractéristiques suivantes :
- possibilité de fonctionnement en continu à des températures de paroi atteignant 1500 °C
- rapport surface d'échange (s) au volume réactionnel (v) très élevé, supérieur à 200 m$^{-1}$ et pouvant atteindre 1000 m$^{-1}$.

Ces deux caractéristiques permettent l'obtention d'une densité de flux thermique très importante à un niveau de température élevé.

- Le carbure de silicium, matériau céramique préférentiellement utilisé dans le cadre de l'invention, présente une neutralité totale vis à vis des produits de craquage formés, alors que ce n'est pas le cas des aciers réfractaires ni même des aciers revêtus de couches de matériaux réfractaires.

Les charges hydrocarbonées utilisables dans le cadre de l'invention sont des charges gazeuses dans les conditions normales de température et de pression, comprenant un pourcentage molaire de méthane compris entre 10 et 99%, par exemple entre 20 % et 99 % et préférentiellement entre 30 et 80 %.

Le reste de la charge peut être constitué par des hydrocarbures aliphatiques, saturés ou non, comprenant un nombre d'atomes de carbone égal ou supérieur à deux, tels que par exemple, l'éthylène, l'éthane, le propane ou le propylène ; d'autres éléments gazeux constitutifs de la charge peuvent être l'azote, le gaz carbonique ou l'oxyde de carbone ou, de préférence l'hydrogène, dont la présence permet de réduire la formation de coke. La proportion molaire d'hydrogène peut être de 1 à 90%.

On peut, en restant dans le cadre de l'invention, rajouter aux charges définies ci-dessus de la vapeur d'eau de dilution ; le rapport pondéral de la vapeur d'eau de dilution à la charge hydrocarbonée est de l'ordre de 0,1 à 1.

- Les fluides caloporteurs utilisables au niveau de la zone de pyrolyse peuvent être tous les fluides thermiquement stables à des températures de l'ordre de 1 200 à 1 500 °C ; on préfère utiliser des fumées de combustion de brûleurs, ou des gaz chauds de récupération provenant d'autres procédés.

- Les fluides utilisables pour refroidir les effluents au niveau de la zone de trempe peuvent être par exemple de l'air, seul ou en mélange avec des fumées de combustion, ou encore de la vapeur d'eau à basse température et sous basse pression.

L'invention sera mieux comprise par la description de quelques modes de réalisation, donnés à titre illustratif mais nullement limitatifs, qui en sera faite ci-après à l'aide des figures annexées :

- la figure 1 représente un mode de réalisation du procédé de l'invention, suivant une coupe longitudinale de l'ensemble réacteur-échangeur de trempe
- la figure 2 représente une coupe d'un élément de l'ensemble de section rectangulaire comportant des canaux unitaires carrés
- la figure 3 représente une perspective éclatée d'un module représentatif de la partie zone de pyrolyse, de section rectangulaire.

Sur la figure 1 on a représenté un ensemble multicanaux comprenant une zone de pyrolyse (A) et une zone de trempe (B). Le mélange constitué par la charge hydrocarbonée à traiter et éventuellement par de la vapeur d'eau est préchauffé, dans une zone de préchauffage non portée sur la figure.

- Cette zone de préchauffage peut être soit de type conventionnel, tel qu'un système par convection, soit être constituée par un système d'échangeur de température de type multicanaux, suivant la technologie décrite pour la zone de pyrolyse, le fluide caloporteur parcourant cette zone de préchauffage pouvant avantageusement être constitué par le fluide caloporteur provenant de la sortie de la zone de pyrolyse. A la sortie de cette zone de préchauffage, le mélange pénètre par la ligne (1) dans la zone de pyrolyse (A) où il est réparti au moyen du distributeur (C) dans la pluralité de canaux réactionnels (D).

La ligne pointillée (M) figure environ la moitié de la longueur de la zone de pyrolyse (A) ; la ligne (2) d'arrivée de fluide caloporteur atteint la zone (A) en un point intermédiaire situé entre (M) et (C) à une distance du début de la zone de pyrolyse (A) représentant 5 à 50 % de la longueur totale de cette zone et de préférence 20 à 40 % de la longueur de cette zone ; ce fluide caloporteur est préférentiellement constitué par des fumées de combustion provenant par exemple d'un brûleur non représenté sur la figure.

Ces fumées pénètrent dans la zone (A) et sont réparties dans la pluralité de canaux de chauffage figurés par (F) ; elles parcourent de préférence ces séries de canaux (F) à contre-courant du mélange réactionnel circulant dans les canaux (D) dans la partie de la zone (A) située entre l'arrivée de fumées (2) et leur sortie supérieure (3), et circulent à co-courant du mélange réactionnel entre l'arrivée (2) et la sortie inférieure de fumées (4).

Les lignes de sortie de fumées (3) et (4) comportent des systèmes de réglage de débit (9 et 10), comme, par exemple des vannes papillons, permettant de régler les débits respectifs des fumées entre les sorties (3) et (4).

Dans le cas particulier où l'on souhaite que l'arrivée de fluide caloporteur s'effectue dès le début de la zone (A), celle-ci peut alors ne pas comporter d'arrivée (2), ou bien celle-ci peut être obturée, l'entrée du fluide s'effectue alors par la ligne (2a) et le parcours du fluide est effectué totalement à co-courant du mélange réactionnel, et sa sortie est faite par la seule ligne (4), la sortie (3) étant alors supprimée.

Dans le cas le plus général décrit plus haut, les fumées sortant par les lignes (3) et (4) sont regroupées et renvoyées par la ligne (5) vers la zone de préchauffage (par convection par exemple). L'obturation des canaux (F) par des dispositifs d'obturation étanches (J) permet de délimiter les zones de pyrolyse (A) et de trempe (B). Le fluide de refroidissement pénètre préférentiellement dans la zone de trempe (B) par la ligne (6) située au tout début de l'échangeur, et circule dans les canaux (F') parallèles aux canaux (D), à co-courant des effluents réactionnels, et sort de l'échangeur par la ligne (7). On peut, le cas échéant, inverser le sens de circulation du fluide de refroidissement qui, dans ce cas, pénètre dans la zone d'échange (B) par la ligne (7) et, après refroidi les effluents réactionnels à contre-courant, sort de (B) par la ligne (6).

Les effluents de la réaction ainsi refroidis sont collectés dans la zone (E) et recueillis par la ligne (8). Suivant la nature des charges traitées, ces effluents peuvent, si nécessaire, subir une seconde trempe par addition directe de fluide froid, suivant un procédé connu. Les traitements ultérieurs des effluents font partie de l'art antérieur et sortent du cadre de la présente invention.

- La figure 2 représente une coupe d'un élément de réacteur de section rectangulaire comportant des canaux unitaires carrés.

Sur cette figure, les canaux réactionnels parcourus par la charge à traiter sont représentés par les rangées de canaux (D) tels que décrits dans la figure 1.

Les canaux parcourus par le fluide caloporteur sont représentés par les rangées hachurées de canaux (F) tels que décrits dans la figure 1.

- La figure 3 représente une perspective éclatée d'un module de la partie zone de pyrolyse (A) de l'ensemble zone de pyrolyse- zone de trempe. Sur cette figure, pour des raisons de clarté, le distributeur de charge (C) décrit dans la figure 1 n'est pas représenté. Les désignations utilisées pour les divers éléments sont celles de la figure 1.

Dans cette figure, le cheminement de la charge à traiter est représenté par les flèches (1) ; la charge pénètre dans la zone de pyrolyse (A), est réparte dans la pluralité de canaux unitaires (D), et traverse longitudinalement la zone de pyrolyse (A) dans ces multicanaux (D) pour sortir de la zone (A) par ces mêmes multicanaux en (11) ; ces multicanaux D se continuent dans la zone de trempe non représentée sur la figure, qui se trouve immédiatement accolée à la zone (A).

Le cheminement suivi par le fluide caloporteur est représenté par les flèches situées face aux orifices (2) ; le fluide caloporteur pénètre dans (A) par les orifices (2) situés dans la première moitié de la zone (A) ; il est réparti dans la pluralité des canaux unitaires (F), une partie de ce fluide parcourt ces canaux à contre-courant de la charge circulant dans les canaux (D) et sort de (A) par les orifices (3) ; l'autre partie de ce fluide circule dans les canaux (F) à co-courant de la charge et sorte de (A) par les orifices (4).

L'exemple qui suit sert à illustrer l'invention.

Il existe, conformément à la présente invention, une forme encore améliorée de mise en oeuvre du procédé.

Selon cette forme de réalisation, on ajoute au gaz préchauffé, contenant du méthane, et constituant le réactif du procédé, l'effluent d'une torche à plasma alimentée par un gaz plasmagène. Il est en effet connu que par passage à travers un arc électrique d'un gaz dit plasmagène on crée un milieu réactionnel particulier, électriquement neutre mais riche en ions, électrons, atomes et/ou molécules excitées. Le gaz plasmagène peut être par exemple l'hydrogène, l'argon, la vapeur d'eau, l'azote, le méthane ou tout autre gaz courant ou le mélange de plusieurs d'entre eux en proportions variables. Ce peut être en particulier tout ou partie du gaz alimentant la zone de pyrolyse.

Selon un mode de réalisation préféré de l'invention, une partie du gaz constituant la charge peut être prélevée, avant ou après réchauffage, de préférence après réchauffage, et envoyée à la torche à plasma. L'effluent de la torche est alors injecté immédiatement au sein du reste du fluide réactif juste avant l'entrée dans la zone de pyrolyse.

On peut utiliser à cet effet de 1 % à 20 % de la charge mais on peut également utiliser une source continue de gaz plasmagène. L'effet de cet ensemencement au moyen de plasma est de favoriser les étapes initiales de la pyrolyse et donc de faciliter grandement la transformation du méthane en hydrocarbures de poids moléculaires plus élevés.

Ainsi, il devient possible de travailler à plus basse température que dans le cas où il n'y a pas d'ensemencement soit, par exemple, à une température moyenne dans le réacteur de pyrolyse d'environ cent degrés inférieure.

Bien que le niveau thermique soit plus bas, le taux de transformation du méthane est maintenu. Par contre la tendance à faire des composés acétyléniques est réduite et la tendance à faire des composés oléfiniques et aromatiques est accrue.

Sous cette réserve, la température moyenne de pyrolyse se situe le plus souvent entre 600 et 1 300 °C. Le but de la trempe est de ramener la température du mélange au-dessous de 400 °C, par exemple vers 100-350 °C ou plus bas.

Par ailleurs on a également découvert dans la présente addition, une forme encore améliorée de mise en oeuvre du procédé.

Selon cette forme de réalisation, dont la mise en oeuvre est aisée, on ajoute au gaz préchauffé, contenant du méthane, et constituant le réactif du procédé, au moins un réactif amorceur choisi dans le groupe formé par l'oxygène, l'ozone et le peroxyde d'hydrogène, dans des proportions convenables par rapport à la quantité de réactif introduite dans la zone de réaction.

Le réactif amorceur est introduit dans le mélange de gaz préchauffé, de préférence en quantité relativement faible par rapport au méthane, avant l'introduction du mélange réactionnel dans la zone de pyrolyse.

Sans vouloir être lié par une quelconque théorie, on peut penser que le réactif amorceur, introduit dans la charge préchauffée de préférence à au moins 300 °C et généralement de 500 à 600 °C, favorise la formation de radicaux, en particulier de radicaux méthyle, et favorise ainsi le démarrage de la réaction de conversion du méthane à plus basse température, ce qui permet d'obtenir un meilleur rendement en produits recherchés : éthylène et aromatiques.

Il n'est ainsi pas souhaitable d'avoir une quantité trop importante de réactif amorceur, car cela risquerait de conduire à la formation d'une quantité importante de radicaux et à la formation excessive de produits secondaires, en particulier d'oxydes de carbons ($CO$, $CO_2$)

La quantité du réactif amorceur, introduite dans le mélange de gaz préchauffé, exprimée en pourcentage d'atome-gramme d'oxygène introduits, par rapport à la quantité de méthane exprimée en mole, sera généralement de 0,01 % à 10 % et de préférence de 0,1 % à 1 %.

Selon un mode de réalisation préféré de l'invention, le réactif amorceur est l'oxygène substantiellement pur (c'est à dire renfermant moins de 1 % en volume d'impureté), ou l'oxygène dilué par un gaz inerte tel que, par exemple l'azote ou l'argon, ou encore un mélange plus complexe de gaz contenant de l'oxygène tel que, par exemple l'air, ou l'air enrichi en oxygène ou l'air dilué par un gaz inerte. Il est également possible d'introduire le réactif amorceur en le diluant préalablement dans une portion de mélange gazeux que l'on veut traiter.

Lorsqu'on utilise de l'ozone celui-ci peut être utilisé sous une forme substantiellement pure ou être dilué dans un gaz comme mentionné ci-dessus pour l'oxygène. On peut par exemple employer un mélange d'oxygène et d'ozone ou de l'air ozonisé.

Lorsqu'on utilise du peroxyde d'hydrogène celui-ci peut être utilisé sous une forme substantiellement pure ou sous une forme diluée. Il est aussi possible d'employer du peroxyde d'hydrogène aqueux, encore appelé parfois eau oxygénée, à condition que la quantité d'eau que l'on introduit alors dans le réacteur reste dans les limites précisées plus haut.

Il est possible de préchauffer le réactif amorceur avant son introduction, par exemple, jusqu'à une température de 150 °C.

L'effet de l'introduction d'au moins un réactif amorceur, est de favoriser grandement la transformation du méthane en hydrocarbures de poids moléculaires élevés. Les quantités introduites étant relativement faibles on ne forme qu'une faible proportion d'oxydes de carbone. Cette légère perte est très largement compensée par l'action bénéfique d'amorçage de la réaction, qui permet ainsi de travailler avec des températures plus basses et des temps de séjour de la charge à traiter dans la zone réactionnelle plus courts.

Ainsi, il devient dans ce cas précis généralement possible de travailler, par exemple, à une température moyenne dans le réacteur de pyrolyse d'environ soixante-dix à environ cent degrés inférieure, par rapport au cas où il n'y a pas d'introduction de réactif amorceur. Le temps de séjour dans la zone réactionnelle peut également être légèrement diminué par l'emploi d'un réactif amorceur.

Toute céramique réfractaire résistant à la température maximum du fluide caloporteur peut être utilisée dans le cadre de la présente invention. A titre d'exemples on peut citer les céramiques suivantes : carbure de silicium, mullite, cordiérite et zircone-mullite.

Exemple 1

Dans cet exemple, on procède à la pyrolyse d'un fuel-gaz de raffinerie provenant d'une unité de cracking catalytique en lit fluidisé. La composition molaire de ce mélange, ayant subi au préalable un lavage destiné à le débarasser de ces composants acides, est la suivante :

| Composé | % molaire |
|---|---|
| $H_2$ | 10 |
| $N_2$ | 12 |
| $CH_4$ | 32 |
| $C_2H_4$ | 15 |
| $C_2H_6$ | 14 |
| $C_3H_6$ | 11 |
| $C_3H_8$ | 6 |

- Ce mélange est fractionné en deux parties après compression sous 30 bars et refroidissement à -100 °C ; la partie de tête (A) représente en volume 57 % du mélange et présente la composition molaire suivante :

| Composé | % molaire |
|---------|-----------|
| $H_2$ | 17,5 |
| $N_2$ | 21,1 |
| $CH_4$ | 56,1 |
| $C_2H_4$ | 5,3 |

Cette fraction est préchauffée à 600 °C et est craquée dans une installation conforme à l'invention, comprenant une zone de pyrolyse multicanaux en carbure de silicium, longue de 3 m et dont les canaux unitaires sont de section carrée de 100 mm². Un fluide caloporteur formé de fumées de combustion de brûleur à 1400 °C est envoyé dans les canaux destinés à cet usage à un débit tel que la température du mélange effluent en sortie de zone de pyrolyse soit de 1200 °C ; le temps de séjour de la charge dans cette zone est de 300 ms.

La zone de trempe est alimentée par de l'air comme fluide réfrigérant. A la sortie de la zone de trempe, les gaz effluents sont à 250 °C.

Par refroidissement à température ambiante et séparation des phases solides liquides et gazeuses, on obtient pour 100 moles de charge les produits suivants :

| Produits | Quantité |
|----------|----------|
| $H_2$ | 52,5 moles |
| $N_2$ | 21,1 moles |
| Noir de carbone | 8,96 g |
| Aromatiques | 44,8 g |
| $CH_4$ | 28,1 moles |
| $C_2H_2$ | 3,69 moles |
| $C_2H_4$ | 13,92 moles |

28 moles de méthane, soit 50 % du méthane introduit ont donc été convertis au cours de ce craquage, avec une sélectivité de 61% pour la formation d'éthylène et de 26 % pour la formation d'acétylène.

Exemple 2

L'exemple suivant illustre les avantages de l'utilisation d'une torche à plasma pour ensemencer une charge riche en méthane.

On traite une charge identique à celle de l'exemple 1

| Composé | % molaire |
|---------|-----------|
| $H_2$ | 17,5 |
| $N_2$ | 21,1 |
| $CH_4$ | 56,1 |
| $C_2H_4$ | 5,3 |

dans un appareil identique.

Ce mélange est préchauffé à 600 °C. Au sortir de la zone de préchauffage on prélève 5 % du flux sortant de l'échangeur de préchauffage et on fait passer cette fraction divisée dans une torche à plasma. Le plasma généré est réinjecté dans le flux gazeux en aval du point de prélèvement, à l'entrée des canaux destinés au passage du réactif à pyrolyser.

En vue de compenser l'endothermicité de la réaction de pyrolyse, on envoie des fumées de combustion à 1 300 °C (soit 100 °C plus bas que dans le cas de l'exemple 1) dans les canaux affectés au fluide de chauffage.

La température de sortie du pyrolyseur s'établit aux environs de 1 100 °C. Le temps de séjour de la charge dans la zone de pyrolyse est de 250 ms soit une durée réduite par rapport à l'exemple de référence.

La trempe est réalisée à l'air comme dans l'exemple de référence de manière à refroidir les gaz jusqu'à 250 °C.

Après refroidissement complet et séparation des phases solide, liquide et gazeuse on obtient pour 100 moles de charge la répartition suivante des produits

| Produits | Quantité |
| --- | --- |
| $H_2$ | 51,5 moles |
| $N_2$ | 21,5 moles |
| Noir de carbone | 6 g |
| Aromatiques | 66 g |
| $CH_4$ | 26,7 moles |
| $C_2H_2$ | 2,0 moles |
| $C_2H_4$. | 15,3 moles |

On a placé en regard dans le tableau I les conditions opératoires et résultats respectivement pour l'exemple de référence et pour l'exemple illustrant le perfectionnement apporté ici.

Tableau 1

| Comparaison des deux essais: sans et avec torche à plasma | | |
| --- | --- | --- |
| | Cas de référence sans torche à plasma | Cas avec torche à plasma |
| Températures moyennes dans la zone de pyrolyse | 950°C | 850°C |
| Temps de séjour | 300 ms | 250 ms |
| Conversion du méthane | 50% | 52,5% |
| Rendements (moles) | | |
| $C_2H_2$ | 25% | 14% |
| $C_2H_4$ | 60% | 67% |
| Aromatiques | 12% | 17% |
| Coke | 3% | 2% |

On relève l'effet bénéfique de l'utilisation de la torche à plasma : maintien de la conversion du méthane malgré une température moyenne de pyrolyse plus basse et un temps de séjour plus court, augmentation de la sélectivité de la transformation du méthane en éthylène ou aromatiques, baisse de la sélectivité en coke et en acétylène, produits ici non recherchés.

Exemple 3

L'exemple suivant illustre les avantages de l'utilisation d'un réactif amorceur introduit dans une charge riche en méthane.
On traite une charge ayant la composition suivante :

| Composé | % molaire |
| --- | --- |
| $CH_4$ | 95 |
| $C_2H_6$ | 5 |

La charge est traitée dans un appareil identique à celui utilisé dans l'exemple 1.
La charge est préchauffée à 600 °C.
Deux essais sont effectués l'un avec introduction d'oxygène et l'autre de manière identique au précédent à l'exception du fait que l'on n'introduit pas d'oxygène.
Dans le premier essai, au sortir de la zone de préchauffage, on introduit dans le mélange, une quantité d'oxygène représentant 0,5 % en mole d'oxygène par rapport au méthane (soit 1 % en atome-gramme d'oxygène par rapport au méthane), à l'entrée des canaux destinés au passage du réactif et en amont du point d'introduction du fluide caloporteur.
En vue de compenser l'endothermicité de la réaction de pyrolyse, on envoie des fumées de combustion à 1350 °C dans les canaux affectés au fluide de chauffage.
Dans le cas de l'essai comparatif, effectué sans addition d'oxygène dans la charge, les fumées de combustion utilisées ont une température de 1425 °C.

La température de sortie de pyrolyseur s'établit aux environs de 1150 °C dans le premier essai avec l'oxygène et aux environs de 1210 °C dans le deuxième essai sans oxygène.

Le temps de séjour de la charge dans la zone de pyrolyse est de 250 millisecondes (ms) pour le premier essai avec oxygène et elle est de 300 millisecondes dans le deuxième essai sans oxygène. La trempe est réalisée à l'air comme fluide réfrigérant, de manière à refroidir les gaz jusqu'à 250 °C.

Après refroidissement complet et séparation des phases solide, liquide et gazeuse on obtient pour 100 moles de charge dans chacun des deux essais la répartition des produits donnée dans le tableau 2 ci-après.

Tableau 2

| Produits | Essai 1 avec introduction d'oxygène | Essai 2 sans introduction d'oxygène |
|---|---|---|
| $H_2$ | 64,33 moles | 62,61 moles |
| CO | 1 mole | 0 |
| Noir de carbone | 4 g | 10 g |
| Aromatiques | 66 g | 58,5 g |
| $CH_4$ | 44 moles | 47,5 moles |
| $C_2H_2$ | 4,46 moles | 7,03 moles |
| $C_2H_4$ | 22,84 moles | 19,06 moles |

Dans le tableau 3 ci-après on donne les conditions opératoires et les résultats pour les deux essais.

Tableau 3

| Conditions | Essai 1 avec introduction d'oxygène | Essai 2 sans introduction d'oxygène |
|---|---|---|
| températures moyennes dans la zone de pyrolyse | 900°C | 975°C |
| temps de séjour | 250 ms | 300 ms |
| conversion du méthane | 53,7% | 50% |
| Rendements (moles) | | |
| $C_2H_2$ | 17,5% | 29,6% |
| $C_2H_4$ | 70,0% | 59,2% |
| Aromatiques | 9,95% | 9,47% |
| Coke | 0,65% | 1,75% |

On relève l'effet bénéfique de l'introduction d'une faible quantité d'oxygène : maintien de la conversion du méthane malgré une température moyenne de pyrolyse plus basse et un temps de séjour plus court, augmentation de la sélectivité de la transformation du méthane en éthylène ou aromatiques, baisse de la sélectivité en coke et en acétylène, produits ici non recherchés.

**Revendications**

1.- Procédé de conversion thermique du méthane en hydrocarbures de poids moléculaires plus élevés dans une zone continue multicanaux en matière céramique, constituée par une pluralité de canaux juxtaposés, les dits canaux formant des rangées de deux types différents en alternance, les canaux des rangées d'un premier type s'étendant sur toute la longueur de la zone et les canaux des rangées du second type étant divisés en au moins une première et au moins une seconde sections non-communicantes entre elles par un cloisonnement intermédiaire, les canaux des rangées du premier type étant non-communicants avec les canaux des rangées du second type, caractérisé en ce que l'on fait circuler un mélange gazeux renfermant un pourcentage molaire de méthane compris entre 10 et 99% dans les canaux des rangées du premier type, en ce que l'on fait circuler un fluide caloporteur dans les canaux de ladite première section des rangées du second type, en ce que l'on fait circuler un fluide de refroidissement dans les canaux de ladite seconde section des rangées du second type et en ce que l'on recueille lesdits hydrocarbures de poids moléculaire plus élevé à l'extrémité des canaux des rangées du premier type.

2.- Procédé selon la revendication 1 dans lequel on introduit le fluide caloporteur dans les canaux de ladite première section perpendiculairement à l'axe de ces canaux, en un point intermédiaire situé à une distance du début de cette section (côté alimentation de la charge) représentant 5 à 50% de la longueur totale des canaux de cette section, et on soutire le fluide caloporteur en partie en amont et en partie en aval dudit point intermédiaire.

3.- Procédé selon la revendication 1 ou 2 dans lequel on fait circuler le fluide de refroidissement dans les canaux de la seconde section à co-courant dudit mélange gazeux circulant dans les canaux des rangées du premier type.

4.- Procédé selon l'une des revendications 1 à 3 dans lequel ledit mélange renfermant du méthane est dilué avec de la vapeur d'eau, le rapport pondéral de la vapeur d'eau au mélange étant d'environ 0,1 à 1.

5.- Procédé selon l'une des revendications 1 à 4, caractérisé en ce qu'on ajoute des espèces activées d'une torche à plasma au mélange gazeux avant l'introduction dudit mélange gazeux dans les canaux des rangées du premier type.

6.- Procédé selon la revendication 5, dans lequel la torche à plasma est alimentée par un gaz choisi dans le groupe formé par l'hydrogène, l'argon, la vapeur d'eau, l'azote et le méthane.

7.- Procédé selon l'une des revendications 1 à 6, caractérisé en ce que l'on introduit dans le mélange gazeux, préalablement chauffé à une température d'au moins 300°C, au moins un réactif amorceur choisi dans le groupe formé par l'oxygène, l'ozone et le peroxyde d'hydrogène, avant l'introduction dudit mélange dans les canaux des rangées du premier type, ledit réactif amorceur étant introduit en une quantité exprimée en pourcentage d'atome-gramme d'oxygène par rapport à la quantité de méthane exprimée en mole de 0,01 à 10%.

8.- Procédé selon la revendication 7 dans lequel la quantité de réactif amorceur est de 0,1 à 1%.

9.- Procédé selon l'une des revendications 1 à 8, dans lequel le mélange gazeux renferme 20 à 99% de méthane.

10.- Procédé selon l'une des revendications 1 à 9, dans lequel le mélange renferme de l'hydrogène.

**Patentansprüche**

1. Verfahren zur thermischen Umsetzung von Methan in Kohlenwasserstoffe mit höheren Molekulargewichten in einer kontinuierlichen Vielkanalzone aus keramischem Material, bestehend aus einer Vielzahl nebeneinander angeordneter Kanäle, wobei diese Kanäle Reihen von zwei abwechselnd unterschiedlichen Typen bilden und die Kanäle der Reihen eines ersten Typs sich über die gesamte Länge der Zone und die Kanäle der Reihen des zweiten Typs in wenigstens einen ersten und wenigstens einen zweiten miteinander nicht in Verbindung stehenden Abschnitt durch eine Zwischentrennwand geteilt sind und die Kanäle der Reihen des ersten Typs nicht in Verbindung mit den Kanälen der Reihen vom zweiten Typ stehen, dadurch gekennzeichnet, daß man ein gasförmiges einen Molprozentanteil Methan zwischen 10 und 99% enthaltendes Gemisch in den Kanälen der Reihen vom ersten Typ strömen läßt, daß man ein Wärmeträgerfluid in den Kanälen des ersten Abschnitts der Reihen vom zweiten Typ strömen läßt, daß man ein Kühlfluid in den Kanälen des zweiten Abschnitts der Reihen vom zweiten Typ strömen läßt und daß man diese Kohlenwasserstoffe höheren Molekulargewichts am Austritt aus den Kanälen der Reihen vom ersten Typ sammelt.

2. Verfahren nach Anspruch 1, dadurch gekennzeichnet, daß man das Wärmeträgerfluid in die Kanäle des ersten Abschnitts senkrecht zur Achse dieser Kanäle an einem Zwischenpunkt einführt, der unter einem Abstand vom Beginn dieses Abschnitts (auf der Speiseseite der Charge) sich befindet, der 5 bis 50% der Gesamtlänge der Kanäle dieses Abschnitts darstellt und daß man das Wärmeträgerfluid zum Teil vor, zum Teil hinter diesem Zwischenpunkt abzieht.

3. Verfahren nach Anspruch 1 oder 2, dadurch gekennzeichnet, daß man das Kühlfluid in den Kanälen des zweiten Abschnitts im Gleichstrom mit diesem gasförmigen Gemisch, das in den Kanälen der Reihen vom ersten Typ strömt, strömen bzw. zirkulieren läßt.

4. Verfahren nach einem der Ansprüche 1 bis 3, dadurch gekennzeichnet, daß dieses Methan enthaltende Gemisch mit Wasserdampf verdünnt wird, wobei das Gewichtsverhältnis des Wasserdampfs zum Gemisch etwa 0,1 bis 1 beträgt.

5. Verfahren nach einem der Ansprüche 1 bis 4, dadurch gekennzeichnet, daß man aktivierte Produkte eines Plasmabrenners einem gasförmigen Gemisch vor dem Einführen dieses gasförmigen Gemisches in die Kanäle der Reihen vom ersten Typ zusetzt.

6. Verfahren nach Anspruch 5, dadurch gekennzeichnet, daß der Plasmabrenner von einem Gas gespeist wird, das aus der durch Wasserstoff, Argon, Wasserdampf, Stickstoff und Methan gebildeten Gruppe gewählt wurde.

7. Verfahren nach einem der Ansprüche 1 bis 6, dadurch gekennzeichnet, daß man in das vorher auf wenigstens 300°C erwärmte gasförmige Gemisch wenigstens ein Starterreagens einführt, welches gewählt ist aus der durch Sauerstoff, Ozon und Wasserstoffperoxid gebildeten Gruppe, bevor dieses Gemisch in die Kanäle der Reihen vom ersten Typ eingeführt wird und wobei dieses Starterreagens in einer Menge eingeführt wird, die ausgedrückt wird als Prozent Grammatom Sauerstoff bezogen auf die Methanmenge, ausgedrückt in Mol von 0,01 bis 10%.

8. Verfahren nach Anspruch 7, dadurch gekennzeichnet, daß die Starterreagensmenge 0,1 bis 1% beträgt.

9. Verfahren nach einem der Ansprüche 1 bis 8, dadurch gekennzeichnet, daß das gasförmige Gemisch 20 bis 99% Methan enthält.

10. Verfahren nach einem der Ansprüche 1 bis 9, dadurch gekennzeichnet, daß das Gemisch Wasserstoff enthält.

**Claims**

1. A process for the thermal conversion of methane into hydrocarbons with a higher molecular weight in a continuous multichannel zone of ceramic material, consisting of a plurality of adjacent channels, said channels forming alternate rows of two different types, the channels of the rows of the first type extending over the entire length of the zone and the channels of the rows of the second type being divided in at least one first and at least one second section, not communicating with one another, by an intermediate partition, the channels of the rows of the first type not communicating with the channels of the rows of the second type, characterized in that a gaseous mixture containing a molar percentage of methane ranging from 10 to 99% is circulated in the channels of the rows of the first type, in that a heat exchange fluid is circulated in the channels of said first section of the rows of the second type, in that a cooling fluid is circulated in the channels of said second section of the rows of the second type and in that said hydrocarbons of higher molecular weights are collected at the end of the channels of the rows of the first type.

2. A process according to claim 1 in which the heat exchange fluid is introduced into the channels of said first section perpendicularly to the axis of these channels, at an intermediate point located at a distance from the beginning of this section (charge feed side) representing 5 to 50% of the total length of the channels of this section, and the heat exchange fluid is withdrawn partly upstream and partly downstream of said intermediate point.

3. A process according to claim 1 or 2 in which the cooling fluid is circulated in the channels of the second section co-current with respect to the said gaseous mixture circulating in the channels of the rows of the first type. ·

4. A process according to one of the claims 1 to 3 in which said mixture containing methane is diluted with steam, the steam to mixture weight ratio being about 0.1 to 1.

5. A process according to one of the claims 1 to 4, characterized in that activated species from a plasma torch are added to the gaseous mixture before said gaseous mixture is introduced into the channels of the rows of the first type.

6. A process according to claim 5, in which the plasma torch is fed by a gas chosen from the group formed by hydrogen, argon, steam, nitrogen and methane.

7. A process according to one of the claims 1 to 6, characterized in that at least one initiator reagent chosen from the group formed by oxygen, ozone and hydrogen peroxide is introduced into the gaseous mixture, previously heated to a temperature of at least 300°C, before introducing said mixture into the channels of the rows of the first type, said initiator reagent being introduced in a quantity expressed in gram-atom oxygen percentage with respect to the quantity of methane expressed in mol of 0.01 to 10%.

8. A process according to claim 7 in which the quantity of initiator reagent is 0.1 to 1%.

9. A process according to one of the claims 1 to 8, in which the gaseous mixture contains 20 to 99% of methane. ·

10. A process according to one of the claims 1 to 9, in which the mixture contains hydrogen.